# EUROPEAN PATENT SPECIFICATION

(11) **EP 1 447 103 B1**
(45) Date of publication and mention of the grant of the patent: **12.10.2005**
(21) Application number: 01274746.5
(22) Date of filing: 20.11.2001
(51) Int. Cl.: A61L 9/02

(54) **METHOD OF DISINFECTING AND SCENTING THE AIR USING ESSENTIAL OILS**
VERFAHREN ZUR DESINFEKTION UND PARFÜMIERUNG VON LUFT MIT ESSENTIELLEN ÖLEN
PROCEDE PERMETTANT DE DESINFECTER ET DE PARFUMER L'AIR A BASE D'HUILES ESSENTIELLES

(43) Date of publication of application: 18.08.2004
(73) Proprietor: Zobele Espana, S.A., 08290 Cerdanyola del Vallés (ES)
(72) Inventor: CORTES BAREA, Jordi, ParcTecnol., 08290 Cerdanyola Del Valles (ES); CASERTA, Andrea, ParcTecnol., 08290 Cerdanyola Del Valles (ES); GARCIA FABREGA, Ruben, ParcTecnol., 08290 Cerdanyola Del Valles (ES)
(74) Representative: Carpintero Lopez, Francisco
(86) International application number: PCT/ES2001/000443
(87) International publication number: WO 2003/043667

(56) References cited:
- WO-A-01/76371
- FR-A- 2 565 109

## Description

### OBJECT OF THE INVENTION

The present invention concerns a method for an air freshener that sterilizes and aromatizes based on the emission of particles to the air of a sterilizing and aromatizing compound based on essential oils, in which the particles are generated by evaporation using a wick indirectly heated on its upper part.

Different compounds based on essential oils with a sterilizing action and their mixtures with glycols are also objects of this invention.

### BACKGROUND OF THE INVENTION

The air in a room or a closed space is often colonized by an excess of microorganisms, some of which can be pathogenic or transmit infections. These microorganisms are often air-born facilitating the possible transmission of infectious problems.

Systems to apply insecticides to the air via evaporation of a liquid insecticide using an absorbent wick submerged in the liquid are known in which the active ingredient of the liquid insecticide evaporates to the atmosphere. In the Invention Patent US 4745705, a method is described for the diffusion of insecticides in the air based on the previously mentioned principle.

Also, patents US 5095647; US 4663315; US 5038394 and US 5290546 can be cited that describe insecticide air diffusion systems without describing the apparatus used to generate the particles.

On the other hand, the British Patent GB A2 194442 describes a fumigator apparatus that can emit a chemical agent with insecticide, sterilizing, deodorant action etc. via a porous wick and indirect heat designed to kill insects or micro-organisms or to deodorize, although this patent does not explicitly describe the method of sterilization or the compositions of the corresponding chemical agent for this application.

In the Patent US 5591395, a method is described to sterilize the air using generators of particles to release sterilizing agents to the atmosphere, such that in this patent 90% of the emitted particles have a limited particle size of around 5 mycra, also limiting the working temperature between 50 and 120°C.

Other documents and patents are known that describe the sterilization of surfaces using liquid formulae or aerosol generators, although the products used to sterilize the air are not described. In this way, methods or formulae of sterilizing action can be cited that use essential oils as active ingredients, described in the Spanish patents ES 2023530 and ES 2143172, and also EP 842604 A1 and WO96/39826.

### DESCRIPTION OF THE INVENTION

The sterilizing and aromatizing air freshener method defined in appended claim 1 is based on emission to the air of particles of a sterilizing composition based on essential oils, in which the particles are generated by evaporation via a wick indirectly heated on its upper part, including the composition of essential oils or their agents of a sterilizing action and their mixtures with specific glycol in varying proportions.

The sterilizing and aromatizing air freshener method can be especially applied in a domestic and institutional environment to sterilize and aromatize the air in rooms or other enclosed spaces and the use of essential oils as sterilizing additives and aromatizers and/or their mixtures with glycols in varying proportions, offer the following advantages compared to other conventional sterilization methods:
- The security profile of essential oils is much higher than that of most non-natural antimicrobial additives. In fact, most essential oils of interest are accepted as aromatic agents or food additives for human or animal consumption.
- Essential oils have intrinsic olfactory properties that provide or enhance the aroma of the sterilizing product, permitting the composition to have aromatizing or air freshener properties without needing to add an additional perfume.

Essential oils can be obtained from cinnamon, tea tree, citronella, lemon grass, thyme, citric fruit, lemons, oranges, aniseed, clove, geranium, rose, mint, lavender, eucalyptus, mint, camphor, sandalwood, cedar and its mixtures, without limiting the essential oils used to the previously described products.

The active agents of the essential oils can be pineol, cinnamaldehyde, cinnamic acid, thymol, citral, citronellal, eugenol, menthol, geraniol, eucalyptol (cineol), cedrol, anethol, limonene, carbacrol and its mixtures, without limiting the active agents to these products.

Preferentially, essential oils for use in the present invention are oils from the tea tree, cinnamon and citronella while the main active agents are pineol, cineol, eugenol, cinnamaldehyde, cinnamic acid, citral and citronellal.

The glycols used as solvents for these essential oils and their active agents are selected from propylene glycol, dipropylene glycol, triethylene glycol and mixtures thereof.

Although the efficacy of glycols as aerial sterilization agents is known, as described in patent US 5591395, it is noteworthy that the use of essential oils in aerial sterilization and its mixtures with specific glycols present a clear advantage compared to the use of glycols alone as sterilizing agents. Numerous literature reviews show that the sterilizing action of alcohols is highly dependent on the environmental conditions of temperature and relative humidity. Therefore, humidities higher than 35-40% reduce and even eliminate the sterilization efficacy. This is due to the high hygroscopy of glycols, that causes them to condense, minimizing the effective amount of agent in vapor state in the air.

The sterilization efficacy of essential oils, or mixtures of these with glycols, is not dependent on conditions of temperature or relative humidity, since these agents have little or no hygroscopy and, therefore, remain in the vapor phase as active agents for air sterilization.

Literature reviews previously mentioned correspond to:
1) Jennings, B.H. et al. (1944) The use of glycol vapors for air sterilization and control of airborne infection.
2) Robertson, O.H. (1943) Sterilization of air with glycol vapors.
3) Lester, William et al. (1949) The rate of bactericidal action of triethylene glycol vapor on microorganisms dispersed into the air in small droplets.
4) Hamburger, Morton et al. (1945) The present status of glycol vapors in air sterilization.
5) Bigg, Edward et al. (1945) Epidemologic observations on the use of glycol vapors for air sterilization.
6) Puck, Theodore T. et al. (1943) The bactericidal action of propylene glycol vapor on microorganisms in air.

Emission of sterilizing and aromatizing particles to the air is done by evaporation of the sterilizing and aromatizing liquid by indirectly heating the upper part of the an upper cell or container that contains the sterilizing and aromatizing liquid in its interior that permits a porous or absorbent wick to be inserted to transport the liquid to the exterior, so that around the opening to the cell there is a heating device, that can correspond to any conventional heating device, such as heating rings or one or several PTC, such that the heating element can be any device provided that a temperature of 100°C or above is achieved, another essential condition for the method of the invention.

In any case, the heating element will be connected via a conductor to an electrical current source, that can be a battery or a domestic connection to the electricity mains supply.

The absorbent wick, however, can be made of any conventional material used for this purpose, preferentially ceramic material, or made from polyester or polyethylene fibers, compressed wood, sintered plastics and even carbon fibers.

Clearly, the efficacy of air sterilization is determined by studying the decrease in microbial contamination compared to the initial situation after having using the sterilizing and aromatizing product.

Experiments have been carried out using different types of essential oil formulae, and the references made can be summarized in the following examples, among others:

### EXAMPLE 1 (comparative)

The formula used is the essential oil of the Australian tea tree (Melaleuca Alternifolia) without solvents.

### Working protocol:

In a 1000 liter room, 7-9 grams of sterilizing and aromatizing formula were diffused by evaporation from polyester fiber wicks indirectly heated on their upper part with a PTC heating device at a working temperature of 100°C. At this moment, a controlled quantity of microbial contamination was introduced. The room was furnished with a bubbling air capture system that can be used to capture a known volume of air. The microorganism and the essential oil diffused in the air were left for a contact time of 1 hour. After this time, 225 liters of air were captured and the number of colonies present was counted. This amount corresponds to the microbial load in the air after the sterilization product has acted on the initial contamination for 1 hour. Also, control plates were placed on the room surfaces to make comparisons with the air counts.

The same experiment is repeated without diffusing essential oils in the room. This serves as a control experiment to quantify the efficacy of the sterilizing and aromatizing product.

The microorganisms to be studied include: S.Epidermis, E. Coli and A. Fumigatus.

The results are as follows:

| MICROORGANISM | % MORTALITY |
|---|---|
| S. epidermis | 95-96% |
| E. Coli | 97-99% |
| A. Fumigatus | 92-93% |

These results clearly reflect an important reduction in the amount of microorganisms present in the air, revealing the high sterilizing potential of the formula.

### EXAMPLE 2 (comparative)

The formula tested in this case is the essential oil of Ceylan Cinnamon leaves without solvents.

### Working protocol:

The same protocol as in Example 1, except that the amount of product evaporated in 7 hours is from 3-3.5 grams

The following results were obtained:

| MICROORGANISM | % MORTALITY |
|---|---|
| S. epidermis | 95-96% |
| E. Coli | 96-99% |
| A. Fumigatus | 100% |

These results once again reflect an important reduction in the amount of microorganisms present in the air, revealing the high sterilizing potential of the formula.

### EXAMPLE 3 (comparative)

The formula tested is in this case the essential oil of Citronnela without solvents.

### Working protocol:

The same as for Example 1, except that the amount of product evaporated in 7 hours is from 5.5-6.5 grams

The results are as follows:

| MICROORGANISM | % MORTALITY |
|---|---|
| S. epidermis | 95-96% |
| E. Coli | 87-95% |
| A. Fumigatus | 90-95% |

These results again reflect an important reduction in the amount of microorganisms present in the air, revealing the high sterilizing potential of the formula.

### EXAMPLE 4

The formula tested in this case is the essential oil of Ceylan cinnamon leaves at 20% with 80% propylene glycol as solvent.

### Working protocol:

The same as for Example 1, except that the amount of product evaporated in 7 hours is from 7-8.5 grams.

The results are as follows:

| MICROORGANISM | % MORTALITY |
|---|---|
| S. epidermis | 98-99% |
| E. Coli | 100% |
| A. Fumigatus | 100% |

These results once again reflect an important reduction in the amount of microorganisms present in the air revealing the high sterilizing potential of the formula.

### EXAMPLE 5

The formula tested in this case is the essential oil of the Ceylan cinnamon leaves at 10% with 90% propylene glycol as solvent.

### Working protocol:

The same as for Example 1, except that the amount of product evaporated in 7 hours is from 7-8.5 grams.

The results are as follows:

| MICROORGANISM | % MORTALITY |
|---|---|
| S. epidermis | 94-97% |
| E. Coli | 100% |
| A. Fumigatus | 100% |

These results once again reflect an important reduction in the amount of microorganisms present in the air, revealing the high sterilization potential of the formula.

### EXAMPLE 6

The formula tested in this case is the essential oil of Citronella at 20% with 80% propylene glycol as solvent.

### Working protocol:

The same protocol as in Example 1, except that the amount of product evaporated in 7 hours is from 5.0-7.0 grams

The results are as follows:

| MICROORGANISM | % MORTALITY |
|---|---|
| S. epidermis | 98-99% |
| E. Coli | 99% |
| A. Fumigatus | 98-100% |

These results once again reflect an important reduction in the amount of microorganisms present in the air, revealing the high sterilization potential of the formula.

### EXAMPLE 7

The formula tested in this case is the essential oil of Citronnela at 10% with 90% propylene glycol as a solvent.

### Working protocol:

The same protocol as for Example 1, except that the amount of product evaporated in 7 hours ranges from 8.0-9.0 grams.

The results are as follows:

| MICROORGANISM | % MORTALITY |
|---|---|
| S. epidermis | 98-99% |
| E. Coli | 95-97% |
| A. Fumigatus | 100% |

These results once again reflect an important reduction in the amount of microorganisms present in the air, revealing the high sterilization potential of the formula.

## Claims

1. Sterilizing and aromatizing air-freshener method based on essential oils, aimed at achieving a high mortality of selected micro-organisms with pathogenic potential or the ability to transmit infections, in the air of a room, or to inhibit microbial growth in the room air as well as serving as an air freshener or aromatizer, wherein particles are diffused into the air of an sterilizing, aromatizing and air freshening composition based on essential oils or their agents and mixtures of these dissolved in propylene glycol, dipropylene glycol, triethylene glycol or mixtures thereof, having generated the product particles by evaporation by indirectly heating the upper part of a wick,
**characterised in that** the working temperature to achieve evaporation of the composition and the corresponding emission of particles is equal to or higher than 100°C.

2. Sterilizing and aromatizing air freshener method based on essential oils according to claim 1 **characterised in that** the essential oils are preferentially obtained from the tea tree, leaves of Ceylan cinnamon and citronnela.

## Patentansprüche

1. Sterilisierendes und aromatisierendes Luftreinigungs- bzw. Luftverbesserungsverfahren auf der Basis von etherischen Ölen, das darauf abzielt, eine hohe Mortalität von ausgewählten Mikroorganismen mit pathogenem Potenzial oder der Fähigkeit zum Übertragen von Infektionen in der Luft eines Raumes zu erreichen oder das Mikrobenwachstum in der Raumluft zu hemmen sowie als Luftreiniger bzw. Luftverbesserer oder Aromatisierer zu dienen, worin Teilchen einer sterilisierenden, aromatisierenden und luftreinigenden bzw. luftverbessernden Zusammensetzung auf der Basis von etherischen Ölen oder ihren Wirkstoffen und Gemischen von diesen, gelöst in Propylenglycol, Dipropylenglycol, Triethylenglycol oder Gemischen davon, in die Luft diffundieren gelassen werden, wobei die Produktteilchen durch Verdampfung durch indirektes Erwärmen des oberen Teils eines Dochtes erzeugt werden, **dadurch gekennzeichnet, dass** die Arbeitstemperatur zum Erreichen einer Verdampfung der Zusammensetzung und der entsprechenden Emission von Teilchen gleich oder höher als 100°C ist.

2. Sterilisierendes und aromatisierendes Luftreinigungs- bzw. Luftverbesserungsverfahren auf der Basis von etherischen Ölen nach Anspruch 1, **dadurch gekennzeichnet, dass** die etherischen Öle vorzugsweise aus dem Teebaum, Blättern von Ceylon-Zimt (Ceylan cinnamon) und Citronella erhalten werden.

## Revendications

1. Procédé de stérilisation et d'aromatisation rafraíchisseur d'air à base d'huiles essentielles, ayant pour but d'obtenir une mortalité élevée de micro-organismes sélectionnés à potentiel pathogène ou ayant la capacité de transmettre des infections, dans l'air d'une pièce, ou d'empêcher la croissance microbienne dans l'air de la pièce ainsi que pour servir de rafraîchisseur d'air ou d'aromatiseur, où des particules sont diffusées dans l'air d'une composition stérilisante, aromatisante et rafraíchisseuse d'air sur la base d'huiles essentielles ou de leurs agents et de mélange de ceux-ci dissous dans du polyéthylène glycol, du dipropylène glycol, du triéthylène glycol ou leur mélange, en ayant formé les particules du produit par évaporation par chauffage indirect de la partie supérieure d'une mèche,
**caractérisé en ce que** la température de travail pour obtenir l'évaporation de la composition et l'émission correspondante de particules est égale ou supérieure à 100°C.

2. Procédé de stérilisation et d'aromatisation rafraíchisseur d'air sur la base d'huiles essentielles selon la revendication 1, **caractérisé en ce que** les huiles essentielles sont de préférence obtenues à partir de l'arbre à thé, des feuilles de cannelle de Ceylan et de citronnelle.
